# EUROPEAN PATENT SPECIFICATION

(11) **EP 0 947 505 B1**
(45) Date of publication and mention of the grant of the patent: **17.07.2002**
(21) Application number: 99400778.9
(22) Date of filing: 30.03.1999
(51) Int. Cl.: C07D 207/26

(54) **Process for preparing optically active 4-hydroxy-2-pyrrolidone**
Verfahren zur Herstellung optisch aktiver 4-Hydroxy-2-pyrrolidone
Procédé de préparation de dérivés optiquement actifs de 4-hydroxy-2-pyrrolidone

(30) Priority: 31.03.1998 JP 10187398
(43) Date of publication of application: 06.10.1999
(73) Proprietor: Takasago International Corporation, Tokyo 144-8721 (JP)
(72) Inventor: Mitsuhashi, Shigeru, Takasago International Corp., Hiratsuka-shi, Kanagawa-ken (JP); Sumi, Kenzo, Takasago International Corporation, Hiratsuka-shi, Kanagawa-ken (JP); Moroi, Takashi, Takasago International Corporation, Hiratsuka-shi, Kanagawa-ken (JP); Sotoguchi, Tsukasa, Takasago International Corp., Hiratsuka-shi, Kanagawa-ken (JP); Miura, Takashi, Takasago International Corporation, Hiratsuka-shi, Kanagawa-ken (JP)
(74) Representative: Uchida, Kenji

(56) References cited:
- EP-A- 0 787 718
- EP-A- 0 829 472
- PELLEGATA R ET AL: "AN IMPROVED SYNTHESIS OF GAMMA-, DELTA-, AND EPSILON-LACTAMS" SYNTHESIS,DE,GEORG THIEME VERLAG. STUTTGART, 1 August 1978 (1978-08-01), pages 614-616, XP000673066 ISSN: 0039-7881
- SANTANIELLO E ET AL: "CHIRAL SYNTHESIS OF A COMPONENT OF AMANITA MUSCARIA, (-)-4-HYDROXYPYRROLIDIN-2-ONE, AND ASSESSMENT OF ITS ABSOLUTE CONFIGURATION" JOURNAL OF CHEMICAL RESEARCH. SYNOPSES,GB,LONDON, 1984, pages 132-133, XP000197370 ISSN: 0308-2342

## Description

This invention relates to a process for preparing optically active 4-hydroxy-2-pyrrolidone useful as a starting material for synthesizing pharmaceuticals.

### BACKGROUND OF THE INVENTION

Optically active 4-hydroxy-2-pyrrolidone is known as an important intermediate for synthesizing carbapenem antibiotics (see unexamined published Japanese patent application JP-A-1-207,266), and an economical process for preparing the compound has been desired.

Conventional processes for preparing optically active 4-hydroxy-2-pyrrolidone include, for example, (1) a process comprising using bread's yeast to reduce a γ-amino-3-oxobutanoic acid ester whose amino group is protected, to obtain an optically active γ-amino-3-hydroxybutanoic acid ester, then hydrolyzing and removing the protecting group, to obtain γ-amino-3-hydroxybutanoic acid (GABOB) (Synthesis, pp. 403-408, 1992), which is then cyclized using hexamethyldisilazane (Synthesis, pp. 614-616, 1978), (2) a process comprising reducing a 4-chloro-3-oxobutanoic acid ester by using bread's yeast to obtain an optically active form of the corresponding alcohol and cyclizing it by the action of ammonia (J. Chem. Res., Synop. pp. 132-133, 1984), (3) a process comprising hydrogenating an N- substituted-4-amino-3-oxobutanoic acid ester asymmetrically in the presence of a catalytic amount of a ruthenium-optically active phosphine complex to obtain an optically active N-substituted-4-amino-3-hydroxybutanoic acid ester, followed by removing the protecting group and cyclizing the resulting deblocked material in alcohol without isolating the deblocked material (JP-A-9-208,558) and (4) a process comprising (i) reacting a 4-halogeno-3-hydroxybutyrate with an alkali metal or alkaline earth metal azide to produce a 4-azido-3-hydroxybutyrate, (ii) hydrogenating in the presence of a catalyst the azido group of said 4-azido-3-hydroxybutyrate to give a 4-amino-3-hydroxybutyrate, (iii) then cyclising said 4-amino-3-hydroxybutyrate (EP-A-0829472).

Known processes for synthesizing racemic 4-hydroxy-2-pyrrolidone include (5) a process comprising hydrolyzing a 4-chloro-3-hydroxy-butanoic acid ester, prepared according to the method described in JP-A-57-183749, to obtain the corresponding carboxylic acid, which is then cyclized according to the method described in the literature (Tetrahedron Letters, Vol. 21, pp. 2443-2446, 1980) and (6) a process comprising protecting the hydroxyl group of a 4-chloro-3-hydroxybutanoic acid ester first by esterifying with a lower carboxylic acid, and then reacting the protected ester with ammonia and cyclizing the product obtained (JP-A-57-183756 and JP-A-61-176564).

In the above-mentioned processes, Process (1), which uses yeast as a means for obtaining an optically active compound by reduction, is disadvantageous for production efficiency in that the solvent required is about 100 times as much as the reaction substrate and that yeast is used 4 times as much as the substrate. This process is also economically disadvantageous because it requires a special reagent in the cyclization process in the course of the process.

Process (2) uses reduction with yeast for obtaining an optically active compound and is therefore disadvantageous for production efficiency. Cyclization of the 4-chloro-3-oxobutanoic acid ester must be carried out by using a large excess of ammonia, which requires a pressure container, and the resulting product is accompanied with considerable amounts of by-products. Cumbersome purification and isolation processes of 4-hydroxy-2-pyrrolidone are therefore indispensable.

In Process (3), expensive 1,1'-carbonyldiimidazole and potassium malonate are reacted with an N-substituted glycine to synthesize an N-substituted-4-amino-3-oxobutanoic acid ester, starting material for asymmetric hydrogenation. Unit cost for the production is therefore high.

Process (4) does not disclose nor suggest hydrogenating a quaternary ammonium salt of a 4-amino-3-hydroxybutyric acid or ester before cyclisation.

Process (5) uses alumina or silica gel as a catalyst for cyclization to obtain a desired compound. But the yield is low and the process is unpractical.

Process (6), in which the hydroxy group of a 4-chloro-3-hydroxybutanoic acid ester is protected and the ester is then reacted with ammonia to obtain a cyclized product, involves complicated steps and is therefore disadvantageous in industrial applications.

### SUMMARY OF THE INVENTION

In the light of the foregoing situation, the inventors of the present invention have conducted extensive studies in search of an efficient and economical process for preparing an optically active 4-hydroxy-2-pyrrolidone and found a practical process for preparing it using inexpensive starting materials through uncomplicated procedures. The present invention comprises the following aspects.

According to a first aspect of the present invention, there is provided a process for preparing an optically active 4-hydroxy-2-pyrrolidone represented by the general formula (3): 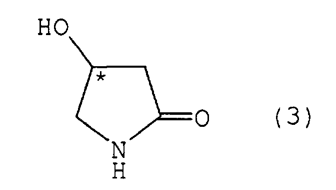 wherein the symbol (*) indicates an asymmetric carbon atom,
the process comprising steps of asymmetrically hydrogenating a substance selected from salts of 4-amino-3-oxobutanoic acid compound represented by the general formula (1) and mixtures thereof: 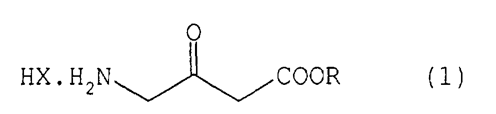 wherein R represents a hydrogen atom or a lower C₁-C₄ alkyl group and X represents a halogen atom, a hydrogensulfate group, a dihydrogenphosphate group, a p-toluenesulfonyloxy group, a benzenesulfonyloxy group, a methanesulfonyloxy group or an acyloxy group,
in the presence of a ruthenium-optically active phosphine complex as catalyst at a molar ratio of 1/100 to 1/10000 under a hydrogen pressure of 0.5 to 10.1 MPa (5 to 100 atm), at a reaction temperature of 10 to 100 °C for 5 to 20 hours, and a lower C₁-C₄ alkanol as solvent, to produce a salt of an optically active 4-amino-3-hydroxybutanoic acid ester represented by the general formula (2): 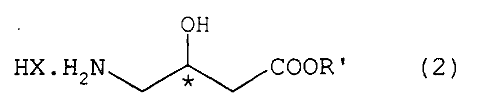 wherein R' represents a lower C₁-C₄ alkyl group the same as, or different from, those represented by R; X represents a halogen atom, a hydrogensulfate group, a dihydrogenphosphate group, a p-toluenesulfonyloxy group, a benzenesulfonyloxy group, a methanesulfonyloxy group or an acyloxy group, and the symbol (*) indicates an asymmetric carbon atom,
followed by a cyclization reaction in the presence of a base at 30 to 70 °C.

In a preferred embodiment, the compound represented by the general formula (1) is a salt of 4-amino-3-oxobutanoic acid;
the substance represented by the general formula (1) is a salt of a 4-amino-3-oxobutanoic acid ester.
the substance represented by the general formula (1) is a salt of a 4-amino-3-oxobutanoic acid ester;
the substance represented by the general formula (1) is a mixture of a salt of 4-amino-3-oxobutanoic acid and a salt of a 4-amino-3-oxobutanoic acid ester; and
the asymmetric hydrogenation reaction of the salts of 4-amino-3-oxobutanoic acid compounds represented by the formula (1) is performed in a solvent represented by the general formula (4):

R"OH (4)

wherein R" represents a lower alkyl group.

### DETAILED DESCRIPTION OF THE INVENTION

The present invention resides in a process for preparing an optically active 4-hydroxy-2-pyrrolidone, which is the objective compound of the present invention and is represented by the formula (3), in a high yield without damaging the optical purity. The process comprises asymmetrically hydrogenating 4-amino-3-oxobutanoic acid compounds represented by the formula (1), in which the amino group is protected in the form of an ammonium salt, in the presence of a catalytic amount of a ruthenium-optically active phosphine complex to produce an ammonium salt of an optically active (3R)- or (3S)-4-amino-3-hydroxybutanoic acid ester represented by the formula (2) at a high enantioselectivity and cyclizing the ammonium salt under heat in an alcohol in the presence of a base.

The present invention will be explained in more detail.

The above 4-amino-3-oxobutanoic acids used as the starting material of the present invention and represented by the formula (1) in which the amino group is protected in the form of an ammonium salt may be synthesized using an easily available 4-halogeno-3-oxobutanoic acid ester as starting material according to, for instance, a method described in J. Am. Chem. Soc., Vol 76, pp. 2887 (1954).

These ammonium salts of 4-amino-3-oxobutanoic acid compounds can be obtained by addition of an excess acid to an azide when the azide is hydrocracked. When these ammonium salts are isolated as a crystal, isopropyl alcohol may be used as a crystallizing solvent. They may also be produced by a process for hydrocracking a benzyl ester of 4-azide-3-oxobutanoic acid used as the substrate in the presence of palladium-carbon in an aqueous hydrochloric acid and 1,4-dioxane as described in Biochemistry, Vol. 36, pp. 1148-1156 (1997).

As 4-amino-3-oxobutanoic acids used as the starting material in the present invention, 4-amino-3-oxobutanoic acid with a hydrogen atom for the substituent R in the formula (1), a 4-amino-3-oxobutanoic acid ester with a lower alkyl group for the substituent R in the formula (1), or a mixture of these may be used.

The lower alkyl group as the substituent in the present invention indicates straight-chain or branched alkyl groups having 1-4-carbon atoms. Examples of the lower alkyl group include a methyl group, ethyl group, propyl group, isopropyl group, n-butyl group, sec-butyl group, isobutyl group, and t-butyl group.

4-amino-3-oxobutanoic acids used as the starting material of the present invention are featured in that the amino group is protected in the form of an ammonium salt. As the acid protecting the amino group of this case, an inorganic acid or an organic acid may be used. Examples of the inorganic acid include hydrochloric acid, sulfuric acid, phosphoric acid, hydrobromic acid and hydroiodic acid. Examples of the organic acid include acetic acid, pivalic acid, p-toluenesulfonic acid, benzenesulfonic acid and methasulfonic acid.

The manufacturing process of the present invention will be explained in detail.

First, when the ammonium salts of 4-amino-3-oxobutanoic acids are asymmetrically hydrogenated, the reaction is carried out in a solvent such as lower alkanols, namely alcohols, e.g., methanol, ethanol, or isopropyl alcohol, in the presence of a catalytic amount of a ruthenium-phosphine complex under a hydrogen pressure of 0.5 to 10.1 MPa (5 to 100 atm) at a reaction temperature of 10 to 100°C for 5 to 20 hours.

Examples of the ruthenium-optically active phosphine complex which can be used as a catalyst for asymmetric hydrogenation include compounds represented by the formula (5) described in JP-B-6-99,367:

RuₓH_{y}Cl_{z}(BINAP)₂Sₚ (5)

wherein BINAP stands for 2,2'-bis(diphenylphosphino)-1,1'-binaphthyl; S represents a tertiary amine; y represents 0 or 1; when y is 0, x represents 2, z represents 4 and p represents 1; and when y is 1, x represents 1, z represents 1 and p represents 0,
compounds represented by the formula (6):

[RuHₗ(BINAP)ᵥ]Y_{w} (6)

wherein BINAP is the same as defined above; Y represents ClO₄-, BF₄- or PF₆-; l represents 0 or 1; when l is 0, v represents 1 and w represents 2; and when l is 1, v represents 2 and w represents 1,
and compounds represented by the formula (7) described in JP-B-7-57,758:

[RuX₁(S)ₘ(BINAP)]Yₙ (7)

wherein BINAP is the same as defined above; S represents benzene which may be substituted with a straight-chain or branched lower alkyl group or carboalkoxy group, or acetonitrile; X is a halogen atom; Y represents a halogen atom, ClO₄-, BF₄-, PF₆- or BPh₄-, wherein Ph represents a phenyl group; l represents 1 or 0; and when l is 1, m represents 1 and n represents 1 and when l is 0, m represents 4 and n represents 2, provided that S is benzene which may have a substituent.

BINAP in these ruthenium-optically active phosphine complexes embraces optical isomers, i.e., (R)-BINAP and (S)-BINAP. An appropriate choice of the isomer is made according to the absolute configuration of a desired compound. That is, (R)-BINAP is to be used for the preparation of an (S)-compound, and (S)-BINAP for an (R)-compound.

The ruthenium-optically active phosphine complex is used at a molar ratio of 1/100 to 1/10,000 and preferably 1/500 to 1/1,000 to the 4-amino-3-oxobutanoic acids (1) as the substrate.

As the lower alkanol used as a solvent for hydrogenation reaction, it is preferable to select the same alcohol as that deriving from the alcohol residue contained in the hydrogenated substrate. The solvent (volume) is usually used 2 to 5 times as much as the substrate (weight).

The process of the present invention enables the reaction to be run without altering the hydrogenation reaction condition even when each of 4-amino-3-oxobutanoic acid and a 4-amino-3-oxobutanoic acid ester which are represented by the formula (1) and used as the starting materials is independently used and even when the both are used in combination with each other.

The ammonium salt of an optically active 4-amino-3-hydroxybutanoic acid ester (2) obtained by the foregoing hydrogenation reaction may remain unisolated while a base, e.g., sodium methylate, sodium ethylate, is added to the hydrogenation reaction solution at room temperature to form a free amine, followed by cyclization reaction, to obtain an optically active 4-hydroxy-2-pyrrolidone represented by the formula (3). The cyclization reaction subsequent to the formation of a free amine is carried out at 30 to 70°C and preferably 30 to 50°C. After the cyclization reaction is completed, the resulting crude product of an optically active 4-hydroxy-2-pyrrolidone is subjected to crystallization and filtration to obtain an optically active 4-hydroxy-2-pyrrolidone of high optical purity.

In the production process according to the present invention, although the reaction process for preparing the objective compound represented by the formula (3) from the starting material represented by the formula (1) involves reaction steps of asymmetric hydrogenation, formation of a free amine and cyclization, it can be carried out in the same container in the same solvent without isolation and purification of intermediate products in the course of the reaction process. Thus the objective compound can be highly yielded in an efficient manner.

### EXAMPLES

The invention will be explained in more detail by way of Reference Examples and Examples which are not intended to be limiting of the present invention.

The following instruments and conditions were adopted as those used for measuring the properties of the compounds prepared in Reference Examples and Examples unless otherwise noted.

### Nuclear Magnetic Resonance Spectrum (¹H-NMR):

1. Model AM-400 (400 MHz) (manufactured by Bruker Inc.)
   - Internal standard:: tetramethylsilane, sodium 3-(trimethylsilyl)-propane sulfonate.
2. Model Gemini 2000 (200 MHz) (manufactured by Varian Inc.)
   - Internal standard:: tetramethylsilane, sodium 3-(trimethylsilyl)-propane sulfonate.

### Melting point:

Model MP-S3 (manufactured by Yanagimoto Syoji K.K.)

### Optical purity:

Hitachi Liquid Chromatography L-6000 (manufactured by Hitachi Ltd.)
- Column:: Chiralpak-AD, 4.6 mm × 250 mm (manufactured by Daicel Chemical Industries, Ltd.)
- Solvent:: hexane/ethanol/methanol = 95/5/3 (by volume)
- Flow rate:: 0.8 ml/min.
- Detection:: 210 nm

### Chemical purity:

Hitachi Liquid Chromatography L-6000 (manufactured by Hitachi Ltd.)
- Column:: Inertsil-ODS-2, 4.6 mm × 250 mm (manufactured by GL Science Ltd.)
- Solvent:: 0.2% aqueous ammonium acetate solution/acetonitrile = 97/3 (by volume)
- Flow rate:: 0.8 ml/min.
- Detection:: 210 nm

### REFERENCE EXAMPLE 1

### Synthesis of methyl 4-azide-3-oxobutanoate

A 1000 ml four-necked flask was charged with 75.3 g (0.5 mol) of methyl 4-chloro-3-oxobutanoate, 450 ml of acetonitrile and 35.8 g (0.55 mol) of sodium azide in flowing nitrogen gas and the mixture was stirred at room temperature (22-24°C) for 20 hours. The produced sodium salt was separated by filtration and the solvent was distilled under reduced pressure. The reaction mixture was washed with 30 g of 5% brine to obtain 77.1 g of a crude product (liquid) of the title compound.

An aliquot of the product was isolated and purified by column chromatography to find that the yield was 95.0% and it contained the title compound in an amount of 74.58 g.
¹H-NMR: δppm (200 MHz, DMSO-d₆)
3.67 (3H, s), 3.68 (2H, s),
4.31 (2H, s)

### REFERENCE EXAMPLE 2

### Synthesis of an ammonium salt of a mixture containing methyl 4-amino-3-oxobutanoate and its carboxylic acid form

A 1000 ml autoclave was charged with 47.1 g of crude product (which contained 45.56 g (0.29 mol) of methyl 4-azide-3-oxobutanoate) prepared in Reference Example 1, 236 ml of methyl alcohol, 65.1 ml of 6N aqueous hydrochloric acid and 0.471 g of 5% palladium-carbon. After the air in the autoclave was replaced by nitrogen three times, the autoclave was charged with hydrogen gas until the hydrogen pressure was raised up to 0.3 MPa (3 atm) to initiate a hydrogenation reaction. A cyclic operation consisting of charging [hydrogen pressure after charging: 0.3 MPa (3 atm)] and purging [hydrogen pressure after purging: 0.01 MPa (0.1 atm)] of hydrogen was repeated 10 times during a period of 5 hours to complete the reaction. The temperature during the reaction was kept between 20 and 25°C. 5% palladium-carbon contained in the reaction solution was filtered through a celite filter. The solvent was then distilled under reduced pressure to obtain 53.2 g of a crude product of the title compound (liquid).

A quantitative measurement using high performance liquid chromatography (HPLC) was made to find that the yield was 85% (methyl ester compound: 73% (a content of 35.5 g), carboxylic acid compound: 12% (a content of 5.35 g)).
¹H-NMR: δpm (200 MHz, DMSO-d₆)
Methyl ester compound:3.68 (3H, s), 3.83 (2H, s),4.04 (2H, s), 8.51 (3H, bs)
Carboxylic acid compound:3.69 (2H, s), 4.04 (2H, s) 8.47 (3H, bs)

### REFERENCE EXAMPLE 3

### Synthesis of an ammonium salt of 4-amino-3-oxobutanoic acid

A 50 ml autoclave was charged with 10.0 g of the crude product (which contained 9.67 g (0.062 mol) of methyl 4-azide-3-oxobutanoate) prepared in Reference Example 1, 48 ml of methyl alcohol, 16 ml of 8N aqueous hydrochloric acid and 0.01 g of 5% palladium-carbon. After the air in the autoclave was replaced by nitrogen three times, the autoclave was charged with hydrogen gas until the hydrogen pressure was raised up to 1 MPa (10 atm) to initiate a hydrogenation reaction. A cyclic operation consisting of charging [hydrogen pressure after charging: 1 MPa (10 atm)] and purging [hydrogen pressure after purging: 0.01 MPa (0.1 atm)] of hydrogen was repeated 10 times during a period of 5 hours to complete the reaction. The temperature during the reaction was kept between 20 and 25°C. 5% palladium-carbon contained in the reaction solution was filtered through a celite filter. The solvent was then distilled under reduced pressure and 30 ml of isopropyl alcohol was then added to the reaction solution. The resulting mixture was crystallized with stirring at 5°C for three hours, followed by filtering, to obtain 5.8 g of the title compound (white crystal). The yield was 61%.

The nuclear magnetic resonance spectrum of this compound was coincident with that obtained in Reference Example 2.

### REFERENCE EXAMPLE 4

### Synthesis of an ammonium salt of methyl 4-amino-3-oxobutanoate

A 500 ml autoclave was charged with 20.0 g of the crude product (which contained 19.35 g (0.123 mol) of methyl 4-azide-3-oxobutanoate) prepared in Reference Example 1, 149 ml of methyl alcohol, 30.45 g (0.16 mol) of p-toluenesulfonic acid one hydrate and 0.2 g of 5% palladium-carbon. After the air in the autoclave was replaced by nitrogen three times, the autoclave was charged with hydrogen gas until the hydrogen pressure was raised up to 0.3 MPa (3 atm) to initiate a hydrogenation reaction. A cyclic operation consisting of charging [hydrogen pressure after charging: 0.3 MPa (3 atm)] and purging [hydrogen pressure after purging: 0.01 MPa (0.1 atm)] of hydrogen was repeated 10 times during a period of 5 hours to complete the reaction. The temperature during the reaction was kept between 20 and 25°C. 5% palladium-carbon contained in the reaction solution was filtered through a celite filter. The solvent was then distilled under reduced pressure to obtain 39.7 g of a crude product of the title compound (liquid).

A quantitative measurement using HPLC was made to find that the yield was 89% and the crude product contained 33.2 g of the title compound.

¹H-NMR: δppm (200 MHz, CDCl₃)
2.31 (3H, s), 3.37 (2H, s), 3.68 (3H, s), 4.04 (2H, s), 7.06 (2H, d, J=8.2Hz),
7.65 (2H, d, J=8.2Hz), 7.85 (3H, bs)

### EXAMPLE 1

### Synthesis of an ammonium salt of methyl (S)-4-amino-3-hydroxybutanoate

A 200 ml autoclave was charged with 27.8 g (0.129 mol) of the crude product (ammonium salt of a mixture of methyl 4-amino-3-oxobutanoate and 4-amino-3-oxobutanoic acid) prepared in Reference Example 2, 84 ml of methanol and 0.218 g (0.258 mmol) of Ru₂Cl₄((R)-BINAP)₂ · NEt₃ in flowing nitrogen gas. The mixture was hydrogenated asymmetrically at a reaction temperature of 50°C under a hydrogen pressure of 3 Mpa (30 atm) for 17 hours (conversion rate: 100%). The solvent was distilled under reduced pressure to obtain 29.6 g of the title compound (liquid).

¹H-NMR: δppm (200 MHz, DMSO-d₆)
2.4-3.0 (4H, m), 3.62 (3H, s), 4.0-4.2 (1H, m), 8.22 (3H, bs)

### EXAMPLE 2

### Synthesis of (S)-4-hydroxy-2-pyrrolidone

A 500 ml four-necked flask was charged with 29.6 g of the ammonium salt of methyl (3S)-4-amino-3-hydroxybutanoate prepared in Example 1, 150 ml of methanol and 29.9 g (0.155 mol) of a methanol solution of 28% sodium methylate and the mixture was reacted at 45 °C for 5 hours.

The produced sodium salt was separated by filtration and the separated sodium salt was washed with 20 ml of methanol. The resulting methanol solution was added to the above methanol solution of the filtrate. The resulting solution, to which 3 g of activated carbon was then added, was stirred at 40°C for 5 hours, followed by filtration. At this time, 20 ml of methanol was used to wash the activated carbon. 70% of methanol was recovered by vacuum distillation of the methanol solution. The residual methanol solution was crystallized with stirring at -15°C for 1.5 hours, followed by filtration. The crystals were washed with a solution of ethyl acetate and methanol (ethyl acetate:methanol=4:1 (by volume)) to obtain 6.85 g of primary crystals. 18 ml of methanol was added to the primary mother liquor weighing 7.1 g to perform crystallization in the same manner as above thereby producing 2.35 g of secondary crystals. The primary and secondary crystals were added up to find that the title compound amounted to 9.2 g and the yield was 70.6%.
Melting point: 158-162°C
Chemical purity: 100%
Optical purity: 100% e.e.
¹H-NMR: δppm (400 MHz, D₂O)
2.26 (1H, dd, J=2Hz, 18Hz), 2.75 (1H, dd, J=6.4Hz, 17.6Hz),
3.32 (1H, dd, J=1.6Hz, 11.6Hz), 3.7 (1H, dd, J=5.2Hz, 11.6Hz),
4.59-4.62 (1H, m)

### EXAMPLE 3

### Synthesis of (S)-4-hydroxy-2-pyrrolidone

A 200 ml autoclave was charged with 10 g (0.0464 mol) of the crude product of the mixture (an ammonium salt of a mixture of methyl 4-amino-3-oxobutanoate and its carboxylic acid form prepared in Reference Example 2), 30 ml of methanol and 0.08 g (0.0926 mmol) of [RuCl(benzene)((R)-BINAP)]Cl in flowing nitrogen gas. The mixture was hydrogenated asymmetrically at a reaction temperature of 50°C under a hydrogen pressure of 3 MPa (30 atm) for 17 hours (conversion rate: 100%). The resultant solution was reacted at 40°C for 5 hours by addition of 24 ml of methanol and 10.76 g (0.056 mol) of a methanol solution of 28% sodium methylate. The produced sodium salt was separated by filtration and the separated sodium salt was washed with 7 ml of methanol. The resulting methanol solution was added to the above methanol solution of the filtrate. The resulting solution, to which 1 g of activated carbon was then added, was stirred at 40°C for 5 hours, followed by filtration. At this time, 7 ml of methanol was used to wash the activated carbon. Methanol amounting to 22 ml was recovered by vacuum distillation of the methanol solution. The residual methanol solution was crystallized with stirring at -15°C for 1.5 hours, followed by filtration. The crystals were washed with a solution of ethyl acetate and methanol (ethyl acetate:methanol=4:1 (by volume)) to obtain 2.24 g of primary crystals. 6 ml of methanol was added to the primary mother liquor weighing 2.41 g to perform crystallization in the same manner as above thereby producing 0.71 g of secondary crystals. The primary and secondary crystals were added up to find that the title compound amounted to 2.95 g and the yield was 63.0%.

This compound was coincident with that obtained in Example 2 in melting point and nuclear magnetic resonance spectrum.

### EXAMPLE 4

### Synthesis of (S)-4-hydroxy-2-pyrrolidone

A 200 ml autoclave was charged with 5 g (0.033 mol) of the ammonium salt of 4-amino-3-oxobutanoic acid prepared in Reference Example 3, 15 ml of methanol and 0.056 g (0.066 mmol) of Ru₂Cl₄((R)-BINAP)₂ • NEt₃ in flowing nitrogen gas. The mixture was hydrogenated asymmetrically at a reaction temperature of 50°C under a hydrogen pressure of 3 MPa (30 atm) for 17 hours (conversion rate: 100%). After the solvent was distilled under reduced pressure, the same procedures as in Example 2 were conducted to obtain 2.78 g of the title compound (yield: 83.4%).

This compound was coincident with that obtained in Example 2 in melting point and nuclear magnetic resonance spectrum.

### EXAMPLE 5

### Synthesis of (S)-4-hydroxy-2-pyrrolidone

The same reaction procedures as in Reference Example 1 were performed, except that ethyl 4-chloro-3-oxobutanoate was used instead of methyl 4-chloro-3-oxobutanoate, to produce ethyl 4-azide-3-oxobutanoate. The resulting ethyl 4-azide-3-oxobutanoate was reacted in the same manner as in Reference Example 2 to produce a crude product (liquid). 11 g of the crude product (containing 7.8 g (0.043 mol) of an ammonium salt of ethyl 4-amino-3- oxobutanoate) was placed in a 200 ml autoclave in flowing nitrogen gas. 33 ml of methanol and 0.078 g (0.086 mmol) of [RuCl((R)-BINAP)]⁺ PF₆- were further added and the mixture was hydrogenated asymmetrically at 50°C under a hydrogen pressure of 3 MPa (30 atm) for 17 hours (conversion rate: 100%). After the solvent was distilled under reduced pressure, the same procedures as in Example 2 were conducted to obtain 2.93 g of the title compound in a yield of 67.3%.

This compound was coincident with that obtained in Example 2 in melting point and nuclear magnetic resonance spectrum.

### EXAMPLE 6

### Synthesis of (S)-4-hydroxy-2-pyrrolidone

A 200 ml autoclave was charged with 36.2 g of the crude product (containing 30.3 g (0.1 mol) of the ammonium salt of methyl 4-amino-3-oxobutanoate) prepared in Reference Example 4, 108 ml of methanol and 0.169 g (0.2 mmol) of Ru₂Cl₄((R)-BINAP)₂•NEt₃ in flowing nitrogen gas. The mixture was hydrogenated asymmetrically at a reaction temperature of 50°C under a hydrogen pressure of 3 Mpa (30 atm) for 17 hours (conversion rate: 100%). After the solvent was distilled under reduced pressure, the same procedures as in Example 2 in the formation of a free amine and the cyclization reaction were conducted to obtain a crude product of (S)-4-hydroxy-2-pyrrolidone. The crude product was subjected to column chromatography using silica gel to obtain 7.3 g of the title compound in a yield of 72.2% from the fraction eluted by an eluent consisting of chloroform and methanol (5:1 by volume).

This compound was coincident with that obtained in Example 2 in melting point and nuclear magnetic resonance spectrum.

### EXAMPLE 7

### Synthesis of (R)-4-hydroxy-2-pyrrolidone

A 200 ml autoclave was charged with 3.62 g of the crude product (containing 3.03 g (0.01 mol) of the ammonium salt of methyl 4-amino-3-oxobutanoate) prepared in Reference Example 4, 10.8 ml of methanol and 0.0169 g (0.02 mmol) of Ru₂Cl₄((S)-BINAP)₂ • NEt₃ in flowing nitrogen gas. The mixture was hydrogenated asymmetrically at a reaction temperature of 50°C under a hydrogen pressure of 3 MPa (30 atm) for 17 hours (conversion rate: 100%). After the solvent was distilled under reduced pressure, the same procedures as in Example 2 in the formation of a free amine and the cyclization reaction were conducted to obtain a crude product of (R)-4-hydroxy-2-pyrrolidone. The crude product was subjected to column chromatography using silica gel to obtain 0.69 g of the title compound in a yield of 68.2% from the fraction eluted by an eluent consisting of chloroform and methanol (5:1 by volume).

This compound was coincident with that obtained in Example 2 in melting point and nuclear magnetic resonance spectrum.

The present invention provides a novel process for preparing an optically active 4-hydroxy-2-pyrrolidone, in which reactions can be performed in one and the same solvent and in one and the same container. The process shows a superior production efficiency and comprises less complicated reaction procedures. Moreover, the present invention can provide an optically active 4-hydroxy-2-pyrrolidone of high optical purity in a higher yield.

## Claims

1. A process for preparing an optically active 4-hydroxy-2-pyrrolidone represented by the general formula (3): 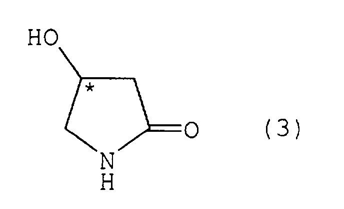 wherein the symbol (*) indicates an asymmetric carbon atom,
the process comprising steps of asymmetrically hydrogenating a substance selected from salts of 4-amino-3-oxobutanoic acid compound represented by the general formula (1) and mixtures thereof: 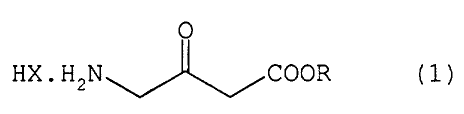 wherein R represents a hydrogen atom or a lower C₁-C₄ alkyl group and X represents a halogen atom, a hydrogensulfate group, a dihydrogenphosphate group, a p-toluenesulfonyloxy group, a benzenesulfonyloxy group, a methanesulfonyloxy group or an acyloxy group,
in the presence of a ruthenium-optically active phosphine complex as catalyst with a molar ratio of 1/100 to 1/10000 under a hydrogen pressure of 0.5 to 10.1 MPa (5 to 100 atm), at a reaction temperature of 10 to 100 °C for 5 to 20 hours, and a lower C₁-C₄ alkanol as solvent, to produce a salt of an optically active 4-amino-3-hydroxybutanoic acid ester represented by the general formula (2): 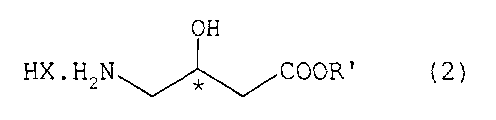 wherein R' represents a lower C₁-C₄ alkyl group the same as, or differing from, those represented by R; X represents a halogen atom, a hydrogensulfate group, a dihydrogenphosphate group, a p-toluenesulfonyloxy group, a benzenesulfonyloxy group, a methanesulfonyloxy group or an acyloxy group, and the symbol (*) indicates an asymmetric carbon atom,
followed by a cyclization reaction in the presence of a base at 30 to 70 °C.

2. A process for preparing an optically active 4-hydroxy-2-pyrrolidone according to Claim 1, wherein said substance represented by the general formula (1) is a salt of 4-amino-3-oxobutanoic acid.

3. A process for preparing an optically active 4-hydroxy-2-pyrrolidone according to Claim 1, wherein said substance represented by the general formula (1) is a salt of a 4-amino-3-oxobutanoic acid ester.

4. A process for preparing an optically active 4-hydroxy-2-pyrrolidone according to Claim 1, wherein said substance represented by the general formula (1) is a mixture of a salt of 4-amino-3-oxobutanoic acid and a salt of a 4-amino-3-oxobutanoic acid ester.

5. A process for preparing an optically active 4-hydroxy-2-pyrrolidone according to Claim 1, wherein said molar ratio is of 1/500 to 1/1000.

6. A process for preparing an optically active 4-hydroxy-2-pyrrolidone according to Claim 1, wherein said lower C₁-C₄ alkanol is methanol, ethanol or isopropyl alcohol.

## Patentansprüche

1. Verfahren zur Herstellung eines optisch aktiven 4-Hydroxy-2-pyrrolidons der allgemeinen Formel (3): 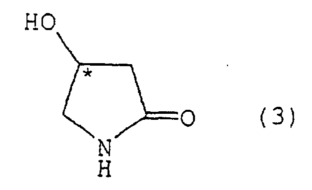 in der das Symbol (*) ein asymmetrisches Kohlenstoffatom bezeichnet,
wobei das Verfahren die Schritte des asymmetrischen Hydrierens einer Substanz, ausgewählt aus Salzen der 4-Amino-3-oxobuttersäure-Verbindung der allgemeinen Formel (1) und Gemischen davon: 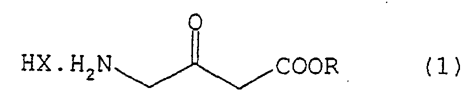 in der R ein Wasserstoffatom oder einen C₁-C₄-Niederalkylrest bedeutet und X ein Halogenatom, eine Hydrogensulfatgruppe, eine Dihydrogenphosphatgruppe, eine p-Toluolsulfonyloxygruppe, eine Benzolsulfonyloxygruppe, eine Methansulfonyloxygruppe oder eine Acyloxygruppe bedeutet,
in Gegenwart eines Ruthenium-optisch aktiven Phosphin-Komplexes als Katalysator mit einem Molverhältnis von 1/100 bis 1/10000 unter einem Wasserstoffdruck von 0,5 bis 10,1 MPa (5 bis 100 atm), bei einer Reaktionstemperatur von 10 bis 100°C über 5 bis 20 Stunden, und eines C₁-C₄-Niederalkanols als Lösungsmittel unter Bildung eines Salzes eines optisch aktiven 4-Amino-3-hydroxybuttersäureesters der allgemeinen Formel (2): 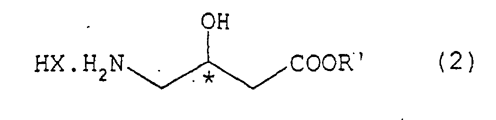 in der R' einen C₁-C₄-Niederalkylrest bedeutet, der gleich denen ist, die durch R dargestellt sind, oder sich davon unterscheidet; X ein Halogenatom, eine Hydrogensulfatgruppe, eine Dihydrogenphosphatgruppe, eine p-Toluolsulfonyloxygruppe, eine Benzolsulfonyloxygruppe, eine Methansulfonyloxygruppe oder eine Acyloxygruppe bedeutet und das Symbol (*) ein asymmetrisches Kohlenstoffatom bezeichnet, gefolgt von einer Cyclisierungsreaktion in Gegenwart einer Base bei 30 bis 70°C umfaßt.

2. Verfahren zur Herstellung eines optisch aktiven 4-Hydroxy-2-pyrrolidons nach Anspruch 1, wobei die Substanz der allgemeinen Formel (1) ein Salz der 4-Amino-3-oxobuttersäure ist.

3. Verfahren zur Herstellung eines optisch aktiven 4-Hydroxy-2-pyrrolidons nach Anspruch 1, wobei die Substanz der allgemeinen Formel (1) ein Salz eines 4-Amino-3-oxobuttersäureesters ist.

4. Verfahren zur Herstellung eines optisch aktiven 4-Hydroxy-2-pyrrolidons nach Anspruch 1, wobei die Substanz der allgemeinen Formel (1) ein Gemisch eines Salzes der 4-Amino-3-oxobuttersäure und eines Salzes eines 4-Amino-3-oxobuttersäureesters ist.

5. Verfahren zur Herstellung eines optisch aktiven 4-Hydroxy-2-pyrrolidons nach Anspruch 1, wobei das Molverhältnis 1/500 bis 1/1000 beträgt.

6. Verfahren zur Herstellung eines optisch aktiven 4-Hydroxy-2-pyrrolidons nach Anspruch 1, wobei das C₁-C₄-Niederalkanol Methanol, Ethanol oder Isopropylalkohol ist.

## Revendications

1. Procédé de préparation d'une 4-hydroxy-2-pyrrolidone optiquement active représentée par la formule générale (3) : 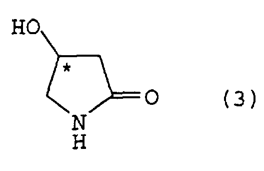 dans laquelle le symbole (*) indique un atome de carbone asymétrique,
le procédé comprenant les étapes consistant à hydrogéner asymétriquement une substance choisie parmi les sels de l'acide 4-amino-3-oxobutanoïque représentés par la formule générale (1) et leurs mélanges : 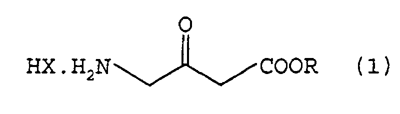 dans laquelle R représente un atome d'hydrogène ou un groupe alkyle C₁-C₄ inférieur et X représente un atome d'halogène, un groupe hydrogénosulfate, un groupe dihydrogénophosphate, un groupe p-toluènesulfonyloxy, un groupe benzènesulfonyloxy, un groupe méthanesulfonyloxy ou un groupe acyloxy,
en présence d'un complexe ruthénium-phosphine optiquement active à titre de catalyseur, avec un rapport molaire de 1/100 à 1/10000 sous une pression d'hydrogène de 0,5 à 10,1 MPa (5 à 100 atm), à une température réactionnelle de 10 à 100°C pendant 5 à 20 heures, et d'un alcanol C₁-C₄ inférieur à titre de solvant, pour produire un sel d'un ester d'acide 4-amino-3-hydroxybutanoïque optiquement actif représenté par la formule générale (2) : 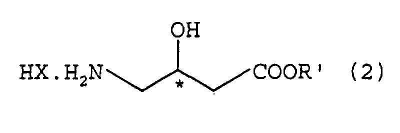 dans laquelle R' représente un groupe alkyle C₁-C₄ inférieur identique ou différent de celui représenté par R ; X représente un atome d'halogène, un groupe hydrogénosulfate, un groupe dihydrogénophosphate, un groupe p-toluènesulfonyloxy, un groupe benzènesulfonyloxy, un groupe méthanesulfonyloxy ou un groupe acyloxy, et le symbole (*) indique un atome de carbone asymétrique,
suivies par une réaction de cyclisation en présence d'une base à 30 à 70°C.

2. Procédé de préparation d'une 4-hydroxy-2-pyrrolidone optiquement active selon la revendication 1, dans lequel ladite substance représentée par la formule générale (1) est un sel d'acide 4-amino-3-oxobutanoïque.

3. Procédé de préparation d'une 4-hydroxy-2-pyrrolidone optiquement active selon la revendication 1, dans lequel ladite substance représentée par la formule générale (1) est un sel d'un ester d'acide 4-amino-3-oxobutanoïque.

4. Procédé de préparation d'une 4-hydroxy-2-pyrrolidone optiquement active selon la revendication 1, dans lequel ladite substance représentée par la formule générale (1) est un mélange d'un sel d'acide 4-amino-3-oxobutanoïque et d'un sel d'un ester d'acide 4-amino-3-oxobutanoïque.

5. Procédé de préparation d'une 4-hydroxy-2-pyrrolidone optiquement active selon la revendication 1, dans lequel ledit rapport molaire est de 1/500 à 1/1000.

6. Procédé de préparation d'une 4-hydroxy-2-pyrrolidone optiquement active selon la revendication 1, dans lequel ledit alcanol C₁-C₄ inférieur est le méthanol, l'éthanol ou l'alcool isopropylique.
